# EUROPEAN PATENT APPLICATION

(11) **EP 4 821 500 A1**
(43) Date of publication of application: **14.10.2026**
(21) Application number: 26170741.8
(22) Date of filing: 03.04.2026
(51) Int. Cl.: A61K 35/28, C12N 5/0775

(54) **ISOLATED OLFACTORY MUCOSA MESENCHYMAL STEM CELLS AND BIOMATERIALS THEREOF FOR PROMOTING PERIPHERAL NERVE REGENERATION**

(30) Priority: 10.04.2025 PT 2025120196
(71) Applicant: Universidade Do Porto, 4099-002 Porto (PT); REQUIMTE - Rede de Química e Tecnologia, 4050-453 Porto (PT)
(72) Inventor: ALVITES, Rui, 4050-313 PORTO (PT); MAURÍCIO, ANA, 4050-313 PORTO (PT); LUÍS, ANA, 4050-313 PORTO (PT); ATAYDE, LUÍS, 4050-313 PORTO (PT); MENDONÇA, CARLA, 4050-313 PORTO (PT)
(74) Representative: Gata-Goncalves, Ligia

(57) **Abstract**

The present invention relates to mesenchymal stem cells isolated from the olfactory mucosa (OM-MSCs) and respective secretome (OMS) integrated with biomaterials such as Neural Guide Conduits/Tubes (NGCs) to promote peripheral nerve regeneration.

Therefore, it is herein disclosed a process of isolating OM-MSCs and the respective secretome, cell lines, secretome and culturing media for said OM-MSCs and OMS.

Also disclosed is a process to obtain biomaterials comprising integrated OM-MSCs and OMS into NGCs, products and uses thereof.

The integration of biomaterials with the cells *per se* or secretome optimizes the microenvironment, enhancing cellular survival, proliferation, and targeted regeneration of nerve tissues.

This invention is in the field of mesenchymal stem cells, biomaterials comprising such cells and peripheral nerve regeneration in humans or animals.

## Description

### Technical field

The present invention relates to mesenchymal stem cells isolated from the olfactory mucosa (OM-MSCs) and respective secretome (OMS) integrated with biomaterials such as Neural Guide Conduits/Tubes (NGCs) to promote peripheral nerve regeneration.

Therefore, it is herein disclosed a process of isolating OM-MSCs and the respective secretome, cell lines, secretome and culturing media for said OM-MSCs and OMS.

Also disclosed is a process to obtain biomaterials comprising integrated OM-MSCs and OMS into NGCs, products and uses thereof.

The integration of biomaterials with the cells *per se* or secretome optimizes the microenvironment, enhancing cellular survival, proliferation, and targeted regeneration of nerve tissues.

This invention is in the field of mesenchymal stem cells, biomaterials comprising such cells and peripheral nerve regeneration in humans or animals.

### Prior art

Peripheral nerve injuries represent an important medical challenge. In addition to the serious consequences associated with the injury itself, which are manifested through motor, sensory and autonomic dysfunctions, traditional specific treatments are generally ineffective and, in most cases, result in disappointing outcomes and permanent functional deficits.

Surgical techniques are traditionally applied, but the effectiveness of these approaches is generally limited in more severe injuries or when surgical intervention is not immediate.

The search for effective and alternative treatments has led to the consideration of new and promising therapies, among which the use of NGCs and cell-based therapies stand out.

**Document Zhu et al. (2024)** discloses the effect of three-dimensional (3D) microcarriers on the biological properties of adipose-derived mesenchymal stem cells (ADSCs). The use of chitosan-based NGCs incorporating ADSC-loaded microcarriers was evaluated to access the enhance of nerve regeneration, demonstrating that this approach improves cell survival within the conduit and promotes better regenerative outcomes. However, the use of ADSCs presents several limitations when compared to OM-MSCs. ADSCs have a more variable differentiation potential and may exhibit reduced neurogenic capacity compared to OM-MSCs, which naturally express neural markers and are derived from a tissue with neurogenic properties. Additionally, the requirement for microcarriers introduces an extra preparation step, making the process more complex and time-consuming than the direct application of cells or even their secretome. Moreover, the success of ADSCs integration within the conduit depends on external factors such as implantation conditions and host response. In contrast, the present invention capitalizes on the regenerative potential of the secretome, eliminating the need for direct cell incorporation and overcoming the limitations associated with ADSCs viability and differentiation, offering a more efficient and scalable approach for peripheral nerve regeneration.

Document WO2023185379A1 describes NGCs loaded with ADSCs to enhance peripheral nerve regeneration. However, the ADSCs have a lower capacity for neurogenic differentiation, their mesodermal origin may determine a lower capacity to integrate into the nervous system of ectodermal origin, they have a greater risk of following an undesirable differentiation in the adipogenic or osteogenic direction and the secretion of growth factors will not be as oriented towards neurogenic differentiation and neuroprotection as in the OM-MSCs.

Document CN109224133A describes a multilayer NGC containing mesenchymal stem cells (MSCs), fabricated through 3D bioprinting using a photocurable hydrogel. This approach enables precise structural customization and controlled cell delivery, but MSC-loaded scaffolds rely on the long-term survival and functionality of the embedded cells, thus not providing a stable and consistent release of bioactive molecules, ensuring sustained neurotrophic and immunomodulatory support without the risks associated with cell viability loss. Furthermore, bio-fabricated conduits require specialized equipment and labour, having strict processing conditions, making large-scale clinical application more challenging. Additionally, bio-imprinted conduits require specific storage conditions to preserve cell viability.

The protocols described in the literature for obtaining OM and OM-MSCs are divided into those applied antemortem and postmortem:
**Girard et al (2011)** described an experimental protocol in which, after confirming the euthanasia of the animal, its decapitation, and the removal of the skin from the head are performed. Then, the jaw is removed, and the facial muscles are eliminated bilaterally. The nasal bones are then removed to expose the nasal turbinates. Finally, the elimination of the turbinates allows the exposure of the nasal septum and the olfactory recess and the collection of the OM. This protocol, although functional, is too time consuming for a task that is intended to be swift. In addition, decapitation of the animal increases the risk of ascending haemorrhage in the upper respiratory tract with contamination of the olfactory recess and olfactory mucosa (OM).
**Tomé et al. (2009) and Ercolin et al. (2016)** have described experimental protocols consisting of performing a longitudinal incision along the skull with posterior removal of the nasal turbinates and access to the olfactory recess and nasal septum. In both cases, the protocols were applied in young animals in which bone fragility allows the described incision without major difficulties. In the case of adult rats, these protocols become more difficult to perform.
**Stamegna et al. (2014)** proposed an alternative experimental protocol for in vivo OM biopsies. Using a stereotactic frame to attach the animal's head, the tissues around the frontal and nasal bones are dissected to allow drilling of the skull in the area just before the cribriform plate, posterior to the nasofrontal suture and anterior to the bregma. Using a forceps, it is then possible to perform the excision of a small OM biopsy.

This method, although relatively quick to collect OM samples in live animals, is too complex for routine use for the isolation and use of OM-MSCs. According to Ito et al. (2019), rhinoscopy using flexible endoscopy is also a possible technique in larger mammals such as dogs and humans, but it requires the availability of specific endoscopy material and the path of the endoscope in the nasal passages can trigger small haemorrhages that can contaminate the nasal cavity and the OM, making it a difficult technique to apply in rats and other small rodents.

The present invention aims to overcome the prior art problems, namely ineffective peripheral nerve regeneration, lack of effective neurotrophic support and limited applicability and customization in current solutions by providing biomaterials based on OM-MSCs isolates and respective secretome having enhanced peripheral nerve regeneration properties.

### Description of the figures

**Figure 1****. Growth Curve and Cell Viability - 35 days assay**
   To determine cell growth curve in function of the total number of cells and their viability at each passage (P), a Trypan blue exclusion cell assay with counting of cells with an automatic counter was performed.
**Fig. 1a** Shows OM-MSCs' growth curve over 35 days, i.e. 15 passages (P15); and
**Fig. 1b** Shows the variation of % cell viability over 35 days i.e. 15 passages (P15).
   From these figures it is possible to observe that the number of cells herein quantified remained stable in the first two passages, with an exponential increase up to the seventh passage.
**Figure 2****. Growth Curve and Cell Viability - 10 days assay**
   OM-MSCs were plated and cultured for 10 days. Then, the Population Doubling Time (PDT) was defined through the formula PDT = CT/PDN, being **CT** the culture time = **10,** and **PDN** the population doubling number; PDN = 3.32(log Xf - log Xi) + S, wherein **Xf** is the number of cells harvested at the end of the subculture, **Xi** is the number of cells used as inoculum and the doubling level of the inoculum used to start the subculture being quantified; further, as the value of **S** is unknown, it was considered as 0. PDT was determined for 3 different moments related to the culture passages, wherein:

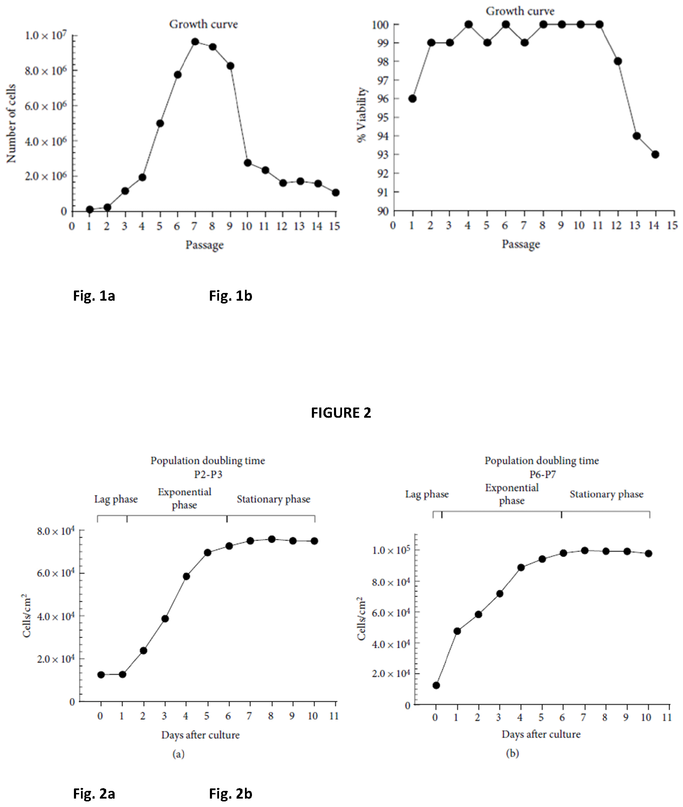

   Fig. 2a shows cells in P2-P3,
   Fig. 2b shows cells in P6-P7, and

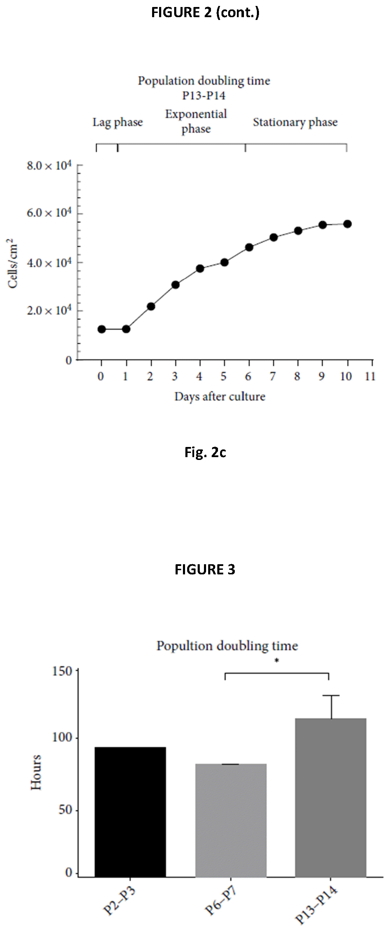

   Fig. 2c shows cells in P13-P14.
   From these figures, it is possible to observe that the growth curve for cells growing in culture had an identical shape for the three determinations of PDT with respect to the exponential and stationary phases. Regarding the lag phase, it was easily identifiable in passages P2-P3 and P13-P14, being not visible in graph P6-P7. The mean value of PDT was 92.87 ± 0.04 hours for the passage P2-P3, 80.94 ± 0.79 hours for P6-P7 and 113.99 ± 18.01 hours for P13-P14.
**Figure 3****. Colony Forming Unit Assays (CFUs)**
   OM-MSCs were plated with a density ranging from 10 to 80 cells in each well of a six-well plate. Cells were incubated for 14 days under standard conditions with daily monitorization to confirm the development of colonies. After this period, culture medium was removed, and wells were stained with Crystal Violet solution for 5-10 min at room temperature.
   The number of colonies in each well was quantified using a magnifying glass. Only visible colonies with more than 1 mm of diameter or with over 50 cells were considered. The clonal efficiency (% of clonogenicity) was calculated using the formula (mean number of colonies counted/total number of seeded cells) ×100.
   CFU assays allowed to confirm the ability of OM-MSCs to generate new fibroblast colonies from single cells. After 14 days of incubation, it was possible to identify an average of 35.33 ± 3.56 colonies with spindle morphology per well, approximately 212 colonies up to 1 mm formed from around 480 seeded cells. The % of clonogenicity was 44.16 ± 4.45%.

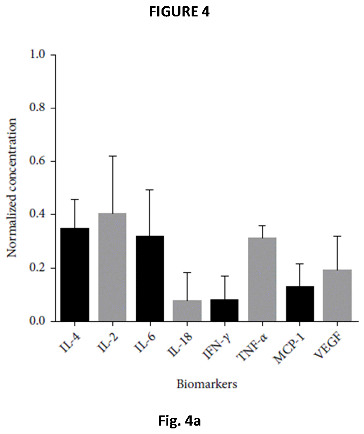

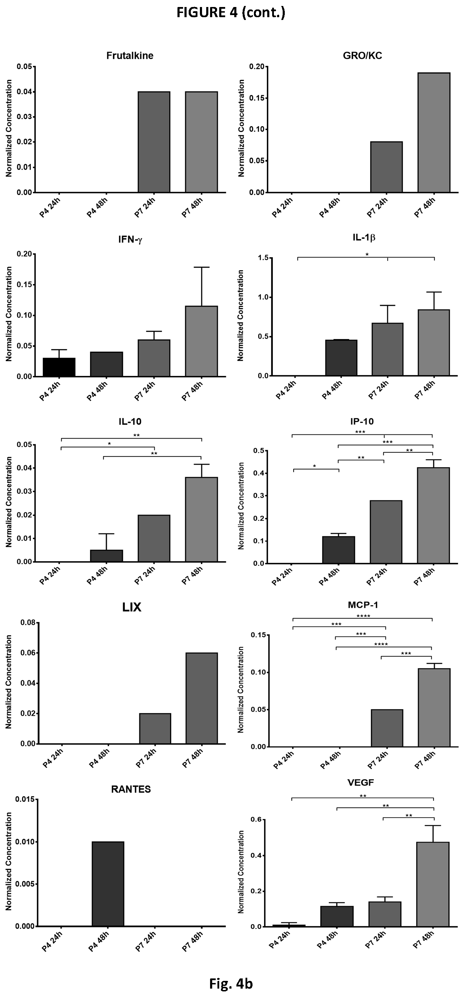

**Figure 4****. OM-MSCs Conditioned Medium / Secretome (OMS) Analysis.**
   To identify specific chemokines and growth factors produced and secreted by OM-MSCs, different conditioned medium (CCM) obtained by sequential cultures were analysed. **OM-MSCs** were cultured until they reached 80-90% confluence and conditioned for 48 hours, with the conditioned medium then collected. The conditioned medium was then analysed by Multiplexing Analysis (Eve Technologies, Canada). The biomarkers identified were grouped into immunomodulatory and immunosuppressive factors (IFN-y and TNF-α), chemokines (MCP-1), growth factors (VGEF), and interleukins (IL).
Fig. 4a shows **OM-MSCs** and OMS in P4,
Fig. 4b shows **OM-MSCs** and OMS in P4 and P7,
   Shows the biomarkers present in the secretome of OM-MSCs. 114 biomarkers were identified at P4 (IL-4, IL-2, IL-6, IL-10, IL-18, IP-10, IFN-y, TNF-α, MCP-1, RANTES, VEGF) and 9 at P7 (IL-1β, IL-10, IP-10, LIX, MCP-1, Frutalkine, GRO/KC, IFN-y, VEGF).
   In most of the biomarkers were identified in both P4 and P7.
   In cases where markers were only identified in one passage, it was usually at P7.
   These figures show that there is an increase in the concentration of biomarkers with increasing conditioning time, which is higher after 48 h than after 24 h of conditioning. In OM-MSCs, the biomarkers Frutalkine, GRO/KC, LIX, and MCP-1 were only identified at passage P7.

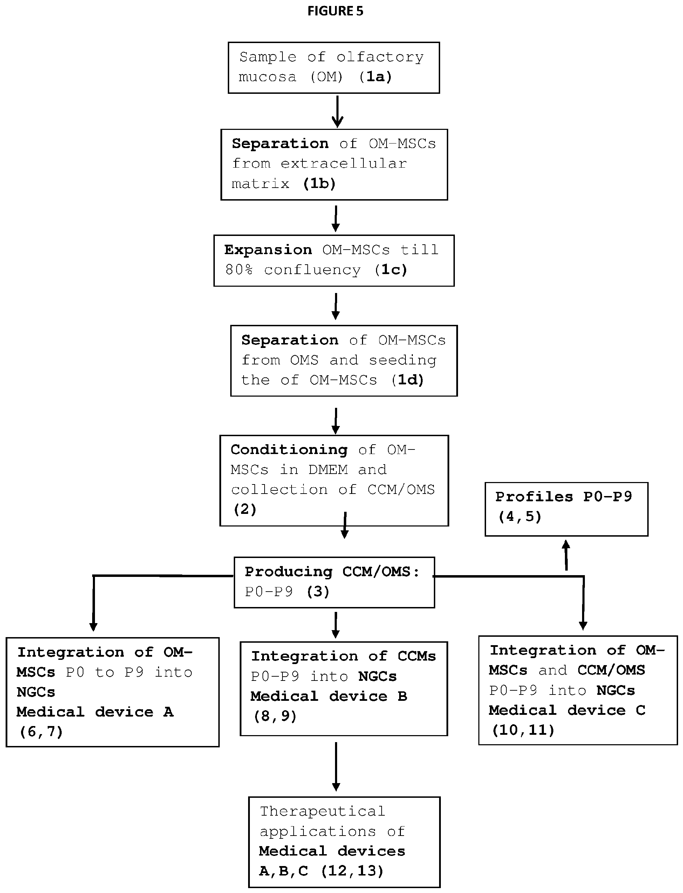

**Figure 5****. Scheme of the process of the invention**
   Fig. 5 describes briefly the main steps of the process of the invention of isolating mesenchymal stem cells from the olfactory mucosa (OM-MSCs), expanding and conditioning said and the respective secretome (OMS) in several passages P0 to P9 cell lines, separation of the conditioned cells from the respective secretome and integration of the several passages of OM-MSCs and OMS in biomaterials, such as Neural Guide Conduits/Tubes (NGCs) to be used in peripheral nerve regeneration therapies, wherein each of the steps is associated with the claims and respective steps.

### Summary of the invention

The present invention relates to biomaterials with integrated isolates of OM-MSCs and their secretome (OMS) for the advanced repair of peripheral nerves after injury.

In one aspect, the present invention is related to a process of obtaining OM-MSCs and the respective OMS according to claim 1.

In a second aspect, the present invention relates to the OM-MSCs and the respective OMS obtained by the above mentioned process, during several passages P0 to P9 according to claim 4.

In another aspect, the present invention relates to the integration of said OM-MSCs and/or OMS in biomaterials for optimization of the microenvironment, enhancing cellular survival, proliferation, and targeted regeneration of nerve tissues, according to claim 6.

The process of the invention allows a faster, less complex procedure and with a lower risk of haemorrhagic contamination than the protocols of the prior art.

Overall, this invention represents a substantial advancement in regenerative medicine, allowing to position it as a cutting-edge solution for peripheral nerve repair with promising implications for both human and veterinary applications, namely:
**Enhanced Nerve Regeneration:** The secretome (OMS) released by OM-MSCs contains a unique profile of neurotrophic and immunomodulatory factors that significantly promote axonal growth, myelination, and overall nerve repair. *In vivo* studies conducted on both rodent and sheep models of peripheral nerve injury indicate that this secretome facilitates a faster recovery process compared to conventional treatment methods.
**Superior Functional Recovery:** Comprehensive assessments have shown substantial improvements in motor performance, sensory functions, and gait patterns in treated animals *in vivo,* showing that OM-MSCs enhance functional recovery following peripheral nerve injury.
**Adaptability for Diverse Applications:** This technology is versatile and can be tailored for application in both human and veterinary medicine, effectively addressing a wide range of peripheral nerve injuries. Established protocols for the isolation and application of OM-MSCs ensure its applicability across multiple species.
**Streamlined Clinical Translation:** The integration of advanced biomaterial technologies, complies with regulatory standards, facilitating an expedited pathway for clinical approval and implementation. The use of clinically relevant models further validates the translational potential of this approach.
**Robust Experimental Validation:** Detailed characterization of OM-MSCs, including their multipotency and high proliferation rate, alongside rigorous functional and histomorphometric evaluations, provides a strong foundation for the therapeutic efficacy of this invention.

### Description of the invention

The present invention relates to isolated OM-MSCs and to their secretome (OMS) in combination with biomaterials in the form of NGCs, to promote peripheral nerve regeneration. Therefore, it is herein disclosed a process of isolating OM-MSCs and the respective secretome, a process to obtain biomaterials comprising NGCs and OM-MSCs and the respective secretome (OMS) and products thereof, including culturing media for said OM-MSCs.

In a first aspect, the present invention relates to a process of preparing and isolating the OM-MSCs and the respective secretome to be used in regenerative therapies, to promote peripheral nerve regeneration.

In a second aspect, the present invention relates to a process of preparing biomaterials with isolated OM-MSCs and the respective secretome to be used in regenerative therapies.

In a third aspect, the present invention relates to medical devices comprising said biomaterials with isolated OM-MSCs and the respective secretome.

### 1. Preparation and isolation of OM-MSCs

### 1.1 Harvesting the OM cells

Harvesting the basal cells olfactory epithelium is performed according to Alvites et al. (2018). Briefly, the OM can be collected under aseptic conditions. Using tweezers, the OM is gently detached from the olfactory recess, nasal septum, and ethmoturbinates, in tissue sample having approx. 1-2 mm², which is transferred to a sterile tube with an adequate culture / maintenance medium, such as phosphate-buffered saline (PBS) and then refrigerated and stored for further use, including the isolation, expansion, and subsequent use of OM-MSCs.

### 1.2. Separation of stem cells from the OM (OM-MSCs)

For the purpose of separation of the mesenchymal stem cells **(OM-MSCs)** from the extracellular matrix of the olfactory mucosa (OM), the collected OM fragments are subjected to enzymatic digestion with trypsin according to the described in Alvites et al. (2020) to promote the separation of the cells and the extracellular matrix, thus resulting in the isolation of respective OM-MSCs.

The OM fragments are placed in direct contact with the surface of the plate. They are then left for a few minutes, such as about 10-15 minutes without culture medium, at room temperature of 18 to 25ºC, to promote their adherence to the plate, thus enhancing the adhesion and maximise the amount of cells able to be successfully isolated.

Confirmation of adhesion can be done by adding base culture medium (BCM) to the wells of the plate, thus corresponding to passage P0.

When the liquid comes into contact with the adhered fragments, no movement is detected. Unsuccessful adhesion provokes the movement of the fragments from their initial position.

### 1.3 Culture and Expansion of OM-MSC

Once adhesion is confirmed as explained in the previous point, culture medium is added to the wells until it completely covers the fragments.

In the scope of the present invention, a suitable culture medium for the intended purposes comprises Dulbecco's modified eagle medium (DMEM/F12), 10% Foetal Bovine Serum (FBS), 1.5% Penicillin/Streptomycin and 1.5% Amphotericin, hereinafter defined simply as base culture medium **(BCM)** and transferred for an incubator in standard culture conditions, such as 37ºC, 5% CO₂, in humidified atmosphere.

After a week (7 days) under these incubation conditions it is possible to observe cells radiating from the explants, thus indicating that the enzymatic digestion is successful, thus the release of the cells from the cellular matrix is attained. Confluency of 70-80% is usually achieved around 4 days after the observation of the first cells radiating from the explant.

Cells were detached from the surface of the plate wells using trypsin to break the bonds of the cells with the surface of the wells, then collected, centrifuged, counted and reseeded in fresh BCM, thus allowing their isolation and subsequent use including using in different passages and cultures.

### 1.4 Conditioning of OM-MSC Cultures

Once 70-80% confluence was reached, the BCM is removed from the well-plates, and the culture is washed with a suitable solution, such as PBS. Subsequently, a Dulbecco's modified eagle medium without antibiotic, antimycotic or FBS (DMEM) is added to the **OM-MSCs,** being these cultures incubated under standard conditions for +/- 48 h until confluency of 70-80% is achieved.

After this period, the culture medium rich in factors secreted by the cells, i.e. the "conditioned culture medium" (CCM-P0) is collected and the supernatant collected and cleaned according to known techniques.

The conditioning process above described can be sequentially repeated, i.e. by first removal of the basal culture medium **(BCM)** of the previous step followed by addition of **DMEM** culture medium, being the resulting **OM-MSCs culture** further incubated under conventional conditions for +/- 48h until confluency of 70-80% is achieved, thus defining a Conditioned Culture Medium **(CCM)** / secretome **(OMS)** corresponding to each of cultivation stages.

In the scope of the present invention, OM-MSCs cultures were sequentially cultivated 9 times (P0 to P9), thus resulting each one of them in cells and secretome P0 to P9, respectively OM-MSCs: P0 to P9 and OMS: P0 to P9.

Because the secretome of each passage (P), corresponds to the conditioned culture medium of the same passage (P), in the scope of the present invention the expressions "conditioned culture medium CCM" and "secretome OMS" can be used interchangeably, so as the corresponding CCMs and OMS of each passages P.

### 2. Characterization of mesenchymal stem cells from the olfactory mucosa and respective secretome

The isolated OM-MSCs are subjected to a set of molecular biology tests and assays to allow their characterization and identification as mesenchymal stem cells.

### 2.1 Growth Curve and Cell Viability

Characterization of the proliferative behaviour of OM-MSCs and determination of their culture maintenance capacity in overtime can be performed by analysis of the growth curve and cell viability.

Typically, the growth curve allows the identification of the initial adaptation phase, the exponential growth phase and the stationary phase, providing essential information about the stability and expansion potential of the cells.

Cell viability allows to ensure that the culture maintained a high number of viable cells, especially over multiple passages. The significance of this evaluation lies in the validation of the proliferative capacity of OM-MSCs, ensuring their viability for applications in regenerative medicine and cell-based therapies, in addition to identifying the ideal number of passages before signs of senescence compromise their functionality.

### 2.2 Population Doubling time

Population Doubling Time (PDT) is an important parameter for assessing the proliferative behaviour of cells in culture, reflecting genetic stability and cell viability throughout passages. PDT can be determined for different moments of culture for evidencing variations in the proliferation rate over time. This analysis allows to determine the best phase for therapeutic application of these cells, ensuring their quality and functionality for potential uses in regenerative medicine.

In the present invention passages (P) P0, and P1 to P14 were evaluated for accessing the characteristics and functional properties of the isolated OM-MSCs and respective secretome (OMS), thus defining CCM-P0 to CCMP-14, wherein CCM-P0 corresponds to the culture medium obtained by addition of base culture medium (BCM) to the culture of isolated stem cells from the olfactory mucosa (OM-MSCs).

Results show that cell proliferation is more efficient in intermediate passages (P6-P7), while early passages present lower adhesion and slower growth; later passages present an increase in PDT, probably due to the onset of cellular senescence phenomena.

### 2.3 Colony Forming Unit Assays

The Colony Forming Unit Assay (CFU) is also an important test to evaluate the clonogenic capacity of cells, indicating their ability to self-renew and proliferate from a single cell. The resulting value allows to validate the quality and potential of these cells in regenerative therapies, as it reflects their stability, expansion capacity and maintenance of essential properties for clinical and experimental applications.

The CFU assay confirmed that OM-MSCs originate new colonies with fibroblastic morphology, presenting a clonogenicity rate of approximately 44.16%.

### 2.4 Differentiation Protocols

Differentiation protocols aiming to evaluate the capacity of mesenchymal stem cells to differentiate into different cell lineages, which is an important criterion for their characterization and potential application in regenerative medicine.

In the present invention, adipogenic, chondrogenic, osteogenic and neurogenic differentiations, according to the minimum criteria established in this area, are disclosed, using specific media to induce differentiation into each cell type and staining techniques to confirm differentiation.

The results demonstrated that OM-MSCs have plasticity and can originate different cell types, reinforcing their applicability in therapies for nerve regeneration and repair.

### 2.5 Reverse Transcriptase Polymerase Chain Reaction

The Reverse Transcriptase Polymerase Chain Reaction (RT-PCR) technique aims to analyse the gene expression of cells, allowing the identification of markers associated with their pluripotency, differentiation and immunophenotypic characteristics.

In the present invention, RT-PCR is used to evaluate the gene expression of OM-MSCs, both in their undifferentiated state and after neurogenic differentiation. This analysis allows the identification of the expression of genes related to pluripotency (such as Sox2), mesenchymal markers (CD73, CD90, CD105) and specific genes of neurogenesis and glial cells (such as GAP43, GFAP and NeuN).

RT-PCR results show that OM-MSCs have cellular plasticity and maintain mesenchymal characteristics throughout the passages, but can be induced to neurogenic differentiation, altering their gene expression profile. Confirmation of these changes by RT-PCR reinforces the viability of these cells for application in nerve regeneration.

### 2.6 Cytogenetic Analysis

Cytogenetic analysis aims to evaluate the chromosomal stability of cells, ensuring their safety for therapeutic applications. This analysis can be performed at different passages of the cell culture to identify changes in chromosome number and possible genetic instability events.

Herein disclosed is the cytogenetic analysis for the passages P5, P8 and P11 with the purpose of evaluating earlier, intermediate and later growing times.

Results show that most cells maintained a normal karyotype (42, XY) at all passages. Although, a slight reduction in the percentage of cells with a normal karyotype was observed in later passages. In addition, the reduction in the mitotic index observed at P11 and concomitant lower proliferative activity, corroborates the occurrence of cellular senescence.

These observations are especially important to validate the viability and safety of OM-MSCs, ensuring that their genetic maintenance throughout culture and their suitability for use in regenerative medicine are maintained.

### 2.7 Immunohistochemical Analysis

Immunohistochemical analysis aims to confirm the phenotypic identity of cells and evaluate their protein expression before and after differentiation.

Results confirm the mesenchymal origin of OM-MSCs by the expression of vimentin and c-Kit, in addition to ruling out endothelial (CD31) and epithelial (Cytokeratin AE1/AE3) characteristics. Immunohistochemistry further demonstrated a significant increase in the expression of neuronal and glial markers after differentiation, with strong immunoreactivity for GFAP (+++), NeuN (+) and GAP43 (++), reinforcing the capacity of OM-MSCs to acquire a neurogenic phenotype. These results corroborate the ones obtained by RT-PCR and highlight the suitability of these cells for applications in neural regeneration therapies.

### 2.8 OM-MSCs' Conditioned Medium Analysis / Secretome (OMS)

Aiming to identify specific chemokines and growth factors produced and secreted by OM-MSCs, when subject to culture conditions with basal culture medium **(BCM),** the resulting secretome (OMS) produced can be analysed by known techniques, such as by Multiplexing LASER Bead analysis. At this stage, the secretome (OMS) corresponds to the passage P0, i.e. when the OM-MSCs are collected after incubation under the conditions described in 1.3.

Herein disclosed are the results of several assays having the respective secretome collected at different times of culture; the respective analysis allows to access the effect of the culturing time in the secretome. Times and passages are chosen to allow to observe said effects both in earlier and later stages of the cultures, thus allowing to learn the senesce effect of the cells to the respective secretome composition.

Once 70-80% confluence was reached, the BCM is removed from the well-plates, and the culture is washed with a suitable solution, such as PBS. Subsequently, a culture medium DMEM 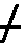 is added to the culture flask, which can be then incubated under standard conditions for +/- 48 h. After this period, the culture medium rich in factors secreted by the cells, i.e. the "conditioned culture medium" (CCM-P0) is collected and the supernatant collected and cleaned according to known techniques, as described in 1.4.

**CCMs** resulting from sequential cultivation better known here as expansion of P0, thus CCM-P1 to CCM-P14 can be stored at refrigeration temperatures, such as -20°C for subsequent use.

According to the present invention, subsequent analysis is performed to search for a specific set of biomarkers, such as immunomodulatory and immunosuppressive factors, chemokines, growth factors, and interleukins.

### 3. Biomaterials and medical devices

Biomaterials comprising integrated OM-MSCs and respective secretome (OMS), according to the present invention into NGCs, products and uses thereof are also encompassed in the scope of the invention.

The integration of biomaterials with the cells *per se* or secretome optimizes the microenvironment, enhancing cellular survival, proliferation, and targeted regeneration of nerve tissues.

Suitable biomaterials include Neural Guide Conduits/Tubes (NGCs), which serve as scaffolds to support and direct axonal regeneration. These conduits can be composed of natural or synthetic polymers, such as chitosan, collagen, fibrin, chitosan, polylactic acid, or polycaprolactone, which provide structural integrity while facilitating cellular adhesion, migration, and differentiation.

The incorporation of OM-MSCs and/or their secretome (OMS) into these biomaterials enhances their regenerative potential by modulating the local microenvironment. This includes the release of neurotrophic factors, cytokines, and extracellular vesicles that promote neuronal survival, reduce inflammation, and stimulate remyelination.

These NGCs can be tailored in terms of porosity, biodegradability, and mechanical properties to suit different applications in peripheral and central nervous system repair. The controlled release of bioactive molecules from the integrated OM-MSCs or OMS further supports tissue regeneration by influencing key cellular processes, such as angiogenesis, neuroprotection, and synaptogenesis.

The present invention, therefore, encompasses products and methods utilizing these biomaterial-integrated NGCs for the treatment of nerve injuries.

### In conclusion:

The present invention discloses biomaterials with OM-MSCs and/or OMS to promote peripheral nerve regeneration.

The innovative approach of using OM-MSCs and/or their secretome integrated in biomaterials such as NGCs to promote peripheral nerve regeneration presents several advantages over the prior art.

Unlike the existing technologies, which may utilize MSCs from other sources or conventional neurotrophic factors alone, the methodology of the present invention harnesses the unique regenerative potential of OM-MSCs, known for their high plasticity and neurotrophic support.

Additionally, the integration of biomaterials with the cells *per se* or secretome optimizes the microenvironment, enhancing cellular survival, proliferation, and targeted regeneration of nerve tissues, thus providing a more effective, biologically aligned method for nerve repair, setting it apart from traditional regenerative techniques.

The combined use of NGCs with OM-MSCs, namely through the implantation of cells in the lumen of the guide tube that surrounds the nerve tops, is the most promising mechanism allowing to explore, combine, and maximize the benefits of each of the individual approaches.

### DEFINITIONS AND ABBREVIATIONS

ADSCs - adipose-derived mesenchymal stem cells,
BCM - basal culture medium also called DMEM/F12,
CFU - Colony forming unit assay,
CCM - conditioned culture medium
CMOEC - suture of the nerve ends to the Reaxon^{®} nerve conduit and administration of OECs CCM,
CMOM - suture of the nerve ends to the Reaxon^{®} nerve conduit and administration of OM-MSCs CCM,
CT - culture time,
DMEM - Dulbecco's modified eagle medium,
DMEM/F12 - Dulbecco's modified eagle medium, 10% Foetal Bovine Serum (FBS), 1.5% Penicillin/Streptomycin and 1.5% Amphotericin, also called as BCM
DMSO - dimethyl-sulfoxide,
ECMOEC - EtE, wrapping with Reaxon^{®} nerve conduit and administration of OECs CCM,
ECMOM - EtE, wrapping with Reaxon^{®} nerve conduit and administration of OM-MSCs CCM,
EDS - analysis and energy-dispersive X-ray spectroscopy,
EEPT - experimental extensor postural thrust,
EIT - experimental intermediate toe spread,
EPL - experimental print length,
EPT - extensor postural thrust,
EtE - end-to-end suture,
EtER - EtE and wrapping with Reaxon^{®} NGC,
EtEROM - EtE, wrapping with Reaxon^{®} NGC, and administration of OM-MSCs suspended in Matrigel^{®},
ETS - experimental toe spread,
FBS - foetal bovine serum,
ITF - intermediate toe spread factor,
M - myelin thickness,
MSCs - mesenchymal stem cells,
N - Number of fibres,
NEPT - normal extensor postural thrust,
NGC-CCM - application of an NGC and secretome produced from OM-MSCs applied inside it,
NGCs - neural guide conduits/tubes,
NIT - normal intermediate toe spread,
NPL - normal print length,
NTS - normal toe spread,
OECs - olfactory ensheathing cells,
OM - olfactory mucosa,
OM-MSCS - mesenchymal stem cells from the olfactory mucosa
OMS - secretome from the olfactory mucosa mesenchymal stem cells
P - passage,
PBS - phosphate-buffered saline,
PDN - population doubling number,
PDT - population doubling time,
PL - print length,
PLF - print length factor,
R - suture of the nerve ends to the Reaxon^{®} NGC,
ROM - suture of the nerve ends to the Reaxon^{®} NGC and administration of OM-MSCs suspended in Matrigel^{®},
RT-PCR - reverse transcriptase polymerase chain reaction,
SEM - scanning electron microscopy,
SFI - sciatic functional index,
SSI - static sciatic index,
TSF - toe spread factor,
UC - uninjured control,
W - week,
WRL - withdrawal reflex latency.

### EXAMPLES

Commercial culture media used in the context of the present invention, and according to the general common knowledge of the skilled person in the art are:
- Gibco^{™} StemPro^{™} Adipogenesis Differentiation Kit,
- Gibco^{™} StemPro^{™} Chondrogenesis Differentiation Kit,
- Gibco^{™} StemPro^{™} Osteogenesis Differentiation Kit,
- Mesenchymal Stem Cell Neurogenic Differentiation Medium,
- Dulbecco's modified eagle medium (DMEM/F12).

The tests involving biomaterials, both *in vitro* and *in vivo,* used guide tubes were purchased from Medovent GmbH and KeriMedical.

### Example 1. Olfactory mucosa harvesting in the rat model

The technique developed for collecting olfactory mucosa is performed as described in Alvites et al. (2018). According to the present invention, the harvesting of the OM in rats was performed after euthanasia of the animal. However, this technique does not require mandatory euthanasia, i.e. it can be performed in vivo under anaesthesia conditions, for example in human or other mammal subjects. With the animal in sternal decubitus, the blade of scissors must be carefully inserted into one of the nostrils and gently pushed until a resistance corresponding to the slit of access to the nasal cavity is felt. At this point, the scissors may be rotated to better fit the internal anatomy of the nostril but should never be forced into the nasal cavity to avoid trauma and bleeding. Once the scissors are well positioned, a cut should be performed carefully, starting at the nostril incision below the alar fold and extending along the lateral wall of the nasal cavity. Nasomaxillary sutures can be used as a guiding line and facilitator for cutting along the bone wall. The same careful procedure should be performed on the contralateral side. Finally, a third cut should be initiated perpendicular to the nasal tubercles, advancing slowly into the nasal cavity along the nasoturbinates, nasal septum, and ethmoturbinates. Once the most caudal limit of the nasal cavity is reached, the nasal bones may be retracted caudally or removed with the aid of tweezers. In this way, the entire floor of the nasal cavity will be exposed, as well as the olfactory recess and respective mucosa, caudally. Once the OM has been exposed, the collection can be started carefully. With a tweezer, and atraumatically, the OM lining the olfactory recess and eventual regions of the nasal septum and remnant ethmoturbinates can be detached and placed immediately within the falcon with transport medium. The OM may be recognized by its gelatinous appearance and differentiated from the adjacent respiratory mucosa by its more yellowish coloration. A needle may be used to gently peel the mucosa from the tip of the tweezers at the time of deposition in PBS. Once the collection is completed, the fragments of OM can be used for the isolation, expansion, and subsequent use of OM-MSCs.

### Example 2. Isolation of stem cells from the olfactory mucosa

The collected olfactory mucosa fragments as described above, were subjected to a mixed method to allow the isolation of olfactory mucosal mesenchymal stem cells (OM-MSCs). According to the method described in Alvites et al. (2020), the fragments were initially cut into small fragments of 1-2 mm² and then subjected to enzymatic digestion with trypsin, in a water bath of approx. 37ºC, for +/- 15 minutes with constant agitation, to allow the separation of the cells and the extracellular matrix. Then, the fragments were transferred to wells of six-well plates and allowed to adhere at room temperature (18 to 25ºC) for 5 to 10 minutes. Once the adhesion of the fragments was confirmed, they were submerged in a suitable culture medium comprising DMEM/F12, 10% FBS, 1.5% Penicillin/Streptomycin and 1.5% Amphotericin, said culture medium hereinafter designated as BCM and transferred for an incubator in standard culture conditions (37ºC, 5% CO₂, humidified atmosphere). After a week, cells were observed radiating from the explants, reaching 70-80% confluency 4 days later. Cells were detached using trypsin, centrifuged, counted and reseeded, thus allowing their isolation and subsequent use in different passages.

### Example 3. Characterization of mesenchymal stem cells from the olfactory mucosa

The isolated OM-MSCs were subjected to a set of molecular biology tests and assays, as described in to allow their characterization and identification as mesenchymal stem cells.

### 3.1 Growth Curve and Cell Viability

Cells were maintained in culture over 35 days to determine their growth curve. Whenever a confluence of 70-80% was reached, a new cell passage was made, resulting in a total number of 15 passages. Each passage was performed by discharge of the existent cultured medium, washing with PBS, cell detachment with Trypsin-EDTA, incubation during 3-5 min under standard conditions, centrifugation and elimination of supernatant.

To determine the number of cells and their viability at each passage, the Trypan blue exclusion cell assay with counting of cells with an automatic counter was performed. The number of cells quantified remained stable in the first two passages, with an exponential increase up to the seventh passage. After this phase, a marked decrease in the number of cells quantified was observed, and this value remained stable until P15.

Cell viability was lower in the first passage. Between passage 2 and passage 12 % viability remained between 99% and 100%. In the last three passages the viability decreases progressively. The analysis of the growth curve and cell viability was relevant to understand the proliferative behaviour and stability of OM-MSCs throughout the passages. The exponential growth phase up to P7 indicates a high proliferative capacity of the cells, thus of major importance for their expansion in therapeutic applications.

The subsequent reduction in the growth rate and the progressive decrease in viability in the last passages (P13-P15) indicates the entry into a state of cellular senescence, characterized by a lower division rate and possible functional alteration. These findings are critical to determine the optimal passage interval for clinical or experimental use, ensuring that OM-MSCs maintain their regenerative potential without compromising their biological quality.

### 3.2 Population doubling time

The OM-MSCs were plated with **BCM** in 10 wells of a 12-well plate. For 10 days, the number of cells from one well per day was determined. At the end of the 10 days, the PDT was defined through the formula:
**PDT = CT/PDN,** being **CT** the culture time, in this case, 10 days and **PDN** the population doubling number.
**PDN** was calculated using the formula **PDN** = **3.32(log Xf - log Xi)** + **S,** being **Xf** the cell harvested at the end of the subculture, **Xi** the cell number used as inoculum and the doubling level of the inoculum used to start the subculture being quantified.

Here, as the value of S is unknown, it was considered as 0. PDT was determined for 3 different moments, namely cells in P2-P3, P6- P7, and P13-P14.

The growth curve for cells growing in culture had an identical shape for the three determinations of PDT with respect to the exponential and stationary phases. Regarding the lag phase, it was easily identifiable in passages P2-P3 and P13-P14, being not visible in the graph P6-P7. The mean value of PDT was 92.87 ± 0.04 hours for the passage P2-P3, 80.94 ± 0.79 hours for P6-P7 and 113.99 ± 18.01 hours for P13-P14.

The determination PDT was greatly relevant to assess the proliferation rate of OM-MSCs throughout the passages, providing information about their stability and therapeutic potential.

The results indicate that the cells present a shorter doubling time in the intermediate passage P6-P7, showing a period of greater proliferation and metabolic activity.

The presence of a more evident adaptation phase, such as the lag phase, in the early P2-P3 and late P13-P14 passages indicates a lower proliferative efficiency in these stages, possibly due to a process of adaptation to culture or the onset of cellular senescence.

The significant increase in PDT in the P13-P14 passage reinforces this hypothesis, indicating a reduction in proliferative capacity and possibly in cellular functionality. These findings are essential to establish the ideal passage interval for therapeutic applications, ensuring that OM-MSCs are used at a stage in which they still have high viability and regenerative capacity.

### 3.3 Colony Forming Unit Assays

OM-MSCs were plated with a density ranging from 10 to 80 cells in each well of a six-well plate. Cells were incubated for 14 days under standard conditions with daily monitorization to confirm the development of colonies. After this period, culture medium was removed, and wells were stained with Crystal Violet solution for 5-10 min at room temperature. The number of colonies in each well was quantified using a magnifying glass. Only visible colonies with more than 1 mm of diameter or with over 50 cells were considered. The clonal efficiency, measured as % of clonogenicity, was calculated using the formula:$\left[mean number of colonies counted/total number of seeded cells\right] \times 100 .$

CFU assays allowed to confirm the ability of OM-MSCs to generate new fibroblast-like colonies from single cells. After 14 days of incubation, it was possible to identify an average of 35.33 ± 3.56 colonies with spindle morphology per well, approximately 212 colonies up to 1 mm formed from around 480 seeded cells. The % of clonogenicity was 44.16 ± 4.45%.

The CFU assay is an important test to evaluate the clonogenic capacity of OM-MSCs, reflecting their ability to self-renew and proliferate from single cells. The results obtained, with a clonogenicity rate of 44.16 ± 4.45%, indicate that almost half of the seeded cells maintained their proliferative potential and formed colonies with fibroblastic morphology. This data confirms the proliferative stability of OM-MSCs and their aptitude for in vitro expansion, ensuring their viability for therapeutic applications. The maintenance of this capacity throughout culture suggests that these cells can be a reliable source for tissue regeneration, especially in tissue engineering and regenerative medicine strategies.

### 3.4 Differentiation Protocols

The OM-MSCs were cultured in selected commercially available culture media for a variable period to stimulate their differentiation into different lineages: adipogenic = 14 days, chondrogenic = 14 days, osteogenic = 21 days, and neurogenic = 5 days.

After this period, the cells in culture were subjected to fixation and specific staining of the differentiations: red-O staining allowed the identification of lipid vacuoles in the cytoplasm (adipogenic differentiation); Alcian Blue allowed the identification of chondrogenic aggregates and extracellular matrix stained blue due to the exposure of the proteoglycans (chondrogenic differentiation); Alizarin Red solution allowed the identification of calcium containing osteocytes and extracellular calcium deposits (osteogenic differentiation); clear morphological alterations were observed in the OM-MSCs that acquired a neuroglial-like shape with development of axon and dendrite-like cell structures (neurogenic differentiation). In this way, the capacity of OM-MSCs to follow different lines of differentiation was confirmed.

The evaluation of the differentiation protocols of OM-MSCs aims to confirm their plasticity and potential for therapeutic applications. The results demonstrated that OM-MSCs were able to differentiate into adipogenic, chondrogenic, osteogenic and neurogenic lineages, as evidenced by specific staining and morphological changes. The acquisition of a neuroglial phenotype, with structures similar to axons and dendrites, reinforces their applicability in the regeneration of the nervous system. These findings are essential in the context of the invention, as they validate the use of OM-MSCs as a versatile source of cells for tissue engineering and regenerative therapies, especially in nerve injuries and repair of specialized tissues.

### 3.5 Reverse Transcriptase Polymerase Chain Reaction

RT-PCR targeting specific genes expressed by OM-MSC was performed under different conditions. In a first assay, it was carried out on undifferentiated OM-MSC cells, in passages 4 and 6. Three genes used as markers for pluripotent stem cells NANOG, Oct4, Sox2, four genes used as MSC markers CD105, CD90, CD73, CD44, two genes used as hematopoietic markers CD45, CD34, and genes used as markers of differentiations: osteogenic BSP, and Runx2, chondrogenic Coll II and Aggrecan, adipogenic Ap-2 and AdipoQ, neurogenic NGF and GDNF, tenogenic Tenomodulin, and musclogenic Desmin were analysed. GAPDH was used as a housekeeping gene.

After the assay, it was possible to confirm the expression, by the OM-MSCs, of pluripotency and stemness genes, genes identifying the cells as MSCs and the absence of hematopoietic markers, as well as the expression of markers associated with the different lines of differentiation. It was also possible to perceive a decrease in gene expression associated with pluripotency in higher passages, with a tendency for increased expression of genes associated with specific differentiations, as shown in **Table 1.**

**Table 1. Effect of increased cell passages on the genetic expression of different markers.**

| **Target Gene** | **P4** | | **P6** | | | | |
|---|---|---|---|---|---|---|---|
| | **Ct average OM-MSCs** | **ΔCt OM-MSCs** | **Ct average OM-MSCs** | **ΔCt OM-MSCs** | **ΔΔCt** | **RQ** | **Regul.** |
| **Nanog** | 35,9 ± 0,9 | 9,7 | 38,2 ± 0,0 | 13,5 | 5,4 | 0,0 | - |
| **Oct4** | 35,3 ± 0,0 | 9,1 | 36,5 ± 0,0 | 11,9 | 4,3 | 0,1 | - |
| **Sox2** | 36,0 + 0,0 | 9,8 | 36,0 ± 0,0 | 11,3 | 3,0 | 0,1 | - |
| **CD105** | 33,9 ± 0,0 | 7,7 | 34,5 ± 0,0 | 9,8 | 3,7 | 0,1 | - |
| **CD90** | 26,2 ± 0,2 | -1,5 | 26,4 ± 0,0 | 1,7 | 3,2 | 0,1 | - |
| **CD73** | 32,9 ± 0,3 | 6,7 | 32,9 ± 0,3 | 8,2 | 3,0 | 0,1 | - |
| **CD44** | 29,4 ± 0,1 | 3,2 | 28,1 ± 0,1 | 3,4 | 1,7 | 0,3 | - |
| **CD45** | nd | nd | nd | nd | nd | nd | nd |
| **CD34** | nd | nd | nd | nd | nd | nd | nd |
| **BSP** | 40,6 ± 0,0 | 14,4 | 35,8 ± 0,0 | 11,1 | -1,8 | 3,5 | |
| **Runx2** | 35,1 ± 0,6 | 8,9 | 31,8 ± 0,0 | 7,1 | -0,3 | 1,2 | N |
| **Coll II** | 36,8 ± 0,0 | 10,6 | 30,3 ± 0,3 | 5,7 | -3,4 | 10,5 | |
| **Aggrecan** | 36,6 ± 0,6 | 10,4 | 31,6 ± 0,4 | 7,0 | -1,9 | 3,8 | |
| **Ap-2** | 33,8 ± 0,4 | 7,6 | 27,8 ± 0,1 | 3,2 | -2,9 | 7,5 | |
| **AdipoQ** | nd | nd | nd | nd | nd | nd | nd |
| **NGF** | 36,9 ± 0,7 | 10,7 | 31,7 ± 0,4 | 7,0 | -2,2 | 4,5 | |
| **GDNF** | 34,4 ± 1,7 | 8,2 | 33,0 ± 0,0 | 8,4 | 1,7 | 0,3 | - |
| **Tenomodulin** | 35,0 ± 0,0 | 8,8 | 35,0 ± 0,0 | 10,4 | 3,0 | 0,1 | - |
| **Desmin** | 36,2 ± 0,0 | 10,1 | 36,3 ± 0,1 | 11,6 | 3,1 | 0,1 | - |

| | | | | | | | |
|---|---|---|---|---|---|---|---|
| **Ct, ΔCt, ΔΔCt** and **RQ** values for all genes under study for P4 and P6; nd = non-defined; Regul = Regulation | | | | | | | |

In a second assay, to identify the expression of specific genes by OM-MSCs undifferentiated and after neurogenic differentiation, a new RT-PCR was performed. 22 genes associated with markers of neuroglial expression were searched. Markers associated with glial cells include Aldh1l1, CD40, Cdh2, Cspg4, GAP43, GFAP, MPZ, NCAM, Nes, Ocln, Olig3, Sox10, and Sox2; neuron associated markers include Ascl1, Dcx, MAP2, NueroD1, Rbfox3, Syn, Cdk5r1, Cript, Tubb3, and again GAP43. β- Actin was used as a housekeeping gene. In the genes identified in the two types of cells, upregulation was observed in practically all of them in neurologically differentiated cells compared to undifferentiated ones. No downregulated genes were observed, demonstrating that after neurogenic differentiation, OM-MSCs increase the expression of genes associated with acquired neuroglial characteristics, as shown in **Table 2.**

**Table 2. Effect of neurogenic differentiation on the genetic expression of different markers.**

| **Target Gene** | **Undifferentiated** | | **Differentiated** | | | | |
|---|---|---|---|---|---|---|---|
| | **Ct average** | **ΔCt** | **Ct average** | **ΔCt** | **ΔΔCt** | **RQ** | **Regulation** |
| **Aldh1l1** | 36.5 + 0.4 | 13.1 | 32.8 + 0.3 | 7.4 | -5.7 | 51.9 | |
| **Ascl1** | 38.6 + 1.8 | 15.2 | 39.5 + 0.7 | 14.1 | -1.1 | 2.2 | |
| **Cd40** | 38.7 + 0.7 | 15.4 | 35.1 + 1.0 | 9.6 | -5.7 | 53.2 | |
| **Cdh2** | 30.1 + 0.4 | 6.8 | 30.1 + 0.6 | 4.7 | -2.1 | 4.2 | |
| **Cdk5r1** | 36.4 + 0.8 | 13,0 | 36.3 + 1.3 | 10.9 | -2.2 | 4.5 | |
| **Cript** | 26.9 + 0.2 | 3.5 | 26.5 + 0.4 | 1,0 | -2.5 | 5.6 | |
| **Cspg4** | 28.5 + 0.3 | 5.1 | 29.9 + 0.2 | 4.4 | -0.7 | 1.6 | N |
| **Dcx** | nd | nd | 39.1 + 0.0 | 13.6 | nd | nd | nd |
| **Gap43** | 37.7 + 1.1 | 14.3 | 39.1 + 1.9 | 13.6 | -0.7 | 1.6 | N |
| **Gfap** | 36.3 + 0.8 | 12.9 | 36.9 + 0.3 | 11.5 | -1.4 | 2.6 | |
| **Map2** | 31.5 + 0.2 | 8.1 | 30.5 + 0.4 | 5.1 | -3,0 | 8.2 | |
| **Mpz** | nd | nd | nd | nd | nd | nd | nd |
| **Ncam** | 34.9 + 0.9 | 11.4 | 35.6 + 2.0 | 10.2 | -1.2 | 2.3 | |
| **Nes** | 33.0 + 0.2 | 9.6 | 33.6 + 0.4 | 8.2 | -1.4 | 2.7 | |
| **Neurod1** | 36.3 + 0.2 | 12.9 | 38.4 + 1.9 | 12.9 | 0.0 | 0.1 | N |
| **Ocin** | 37.6 + 1.2 | 14.3 | nd | nd | nd | nd | nd |
| **Olig3** | 40.7 + 0.0 | 17.3 | nd | nd | nd | nd | nd |
| **Rbfox3** | nd | nd | 37.7 + 0.0 | 12.3 | nd | nd | nd |
| **Sox10** | 36.8 + 1.6 | 13.4 | 37.2 + 1.8 | 11.8 | -1.6 | 3,0 | |
| **Sox2** | 36.3 + 0.0 | 12.9 | 36.1 + 0.0 | 10.7 | -2.2 | 4.7 | |
| **Syp** | nd | nd | 36.4 + 1.2 | 11,0 | nd | nd | nd |
| **Tubb3** | 29.8 + 0.5 | 6.4 | 29.4 + 0.7 | 4,0 | -2.4 | 5.4 | |

| | | | | | | | |
|---|---|---|---|---|---|---|---|
| **Ct, ΔCt, ΔΔCt,** and **RQ** values for all genes for OM-MSCs undifferentiated and after neurogenic differentiation. N= normal; ↑ = upregulated; ↓ = downregulated; nd = non-defined. | | | | | | | |

### 3.6 Cytogenetic Analysis

For evaluation of the karyotype, OM-MSCs in three different passages P5, P8 and P11 were submitted to cytogenetic analysis to determine chromosomal stability in terms of chromosome number and occurrence of neoplastic changes. Cytogenetic evaluation allowed the identification of a high percentage of cells with a normal karyotype for the species 42-XY, for all passages although a small decrease in this percentage was observed in passages P8 and P11 compared to passage P5. Regarding the mitotic index, the values are normal for passages P5 and P8 and low for passage P11, as shown in **Table 3.**

**Table 3. Percentage of normal karyotypes and mitotic indexes of OM-MSCs in P5, P8, and P11.**

| **Passage** | **% Cells with normal karyotype (42, XY)** | **Mitotic Index** |
|---|---|---|
| **P5** | 85% | Normal |
| **P8** | 62% | Normal |
| **P11** | 58,80% | Low |

### 3.7 Immunohistochemical Analysis

Two Immunohistochemical assays were performed. In the first one, early passage P5 of OM-MSCs was submitted to immunohistochemical analysis to detect specific antigens. The antibodies were selected to confirm the mesenchymal origin of OM-MSCs vimentin and c-Kit, to discard an endothelial CD31 and epithelial (Cytokeratin AE1/AE3) origin and to identify the expression of neural synaptophysin and glial markers (GFAP). OM-MSCs showed weak immunoreactivity for synaptophysin (comprising about 5% of cell population), moderate for c-kit (comprising 5-80%) and strong staining for GFAP and Vimentin (involving >80% of the cells). Cells were negative for AE1/AE3 and CD31 immunomarkers.

In the second assay, early passage P6 OM-MSCs were submitted to immunohistochemical analysis to detect specific antigens associated with neurogenic differentiation. The antibodies were selected to identify the presence of markers of neuronal (NeuN and GAP43) and glial (GFAP) origin. After neurogenic differentiation, OM-MSCs showed immunoreactivity for GFAP (+++), NeuN (+), and GAP43 (++). In undifferentiated cells, only immunoreactivity for GFAP (+) was observed. These results confirm that after being subjected to neurogenic differentiation, not only do the cells acquire a neurogenic-like morphology but they also undergo changes in the expression of specific neuroglial markers.

### Example 4. Characterization of the OM-MSCs Secretome (OMS)

To identify specific chemokines and growth factors produced and secreted by OM-MSCs, CCM was analysed. In a first assay, for the conditioning, OM-MSCs in early passage P4 were applied. Cells were cultured until they reached 80-90% confluence and conditioned for 48 hours, with the CCM then collected. The CCM was then analysed by Multiplexing Analysis (Eve Technologies, Canada). The biomarkers under study are grouped into immunomodulatory and immunosuppressive factors IFN-y and TNF-α, chemokines MCP-1, growth factors (VGEF), and interleukins IL. All biomarkers were identified in the CCM, as shown in **Table 4.**

**Table 4. Mean normalized concentration of each biomarker in the CCM of OM-MSCs at P4 (mean ± SEM).**

| **Biomarker** | **Mean normalized concentration** |
|---|---|
| **IL-4** | 0,34 ± 0,11 |
| **IL-2** | 0,4 ± 0,22 |
| **IL-6** | 0,32 ± 0,3 |
| **IFNγ** | 0,07 ± 0,1 |
| **IL-18** | 0,08 ± 0,10 |
| **MCP-1** | 0,13 ± 0,9 |
| **TNF-α** | 0,31 ± 0,04 |
| **VEGF** | 0,19 ± 0,13 |

In a second assay, to compare changes in secretory capacity over time, cells in two different passages (P4 and P7) were subjected to the same conditioning and multiplexing analysis process. The list of searched biomarkers includes Eotaxin, Epidermal Growth Factor (EGF), Fractalkine, Interferon Gamma (IFN-y), Interleukins (IL) IL-1α, IL-1β, IL-2, IL-4, IL-5, IL-6, IL-10, IL-12p70, IL- 13, IL-17A, IL-18, IP-10, Human Growth-regulated oncogene/Keratinocyte Chemoattractant (GRO/KC), Tumour Necrosis Factor Alpha (TNF-α), Granulocyte Colony Stimulating Factor (G-CSF), Granulocyte-macrophage Colony stimulating Factor (GM-CSF), Monocyte Chemoattractant Protein-1 (MCP-1), Leptin, LIX, Macrophage Inflammatory Protein (MIP) MIP-1α, MIP-2, Regulated on Activation, Normal T Cell Expressed and Secreted (RANTES), and vascular endothelial growth factor (VEGF). In the secretome of OM-MSCs, 10 biomarkers were identified: Frutalkine, GRO/KC, IFN-y, IL-18β, IL-10, IP-10, LIX, MCP-1, Rantes, and VEGF. In most biomarkers, they were identified in both P4 and P7. In cases where markers were only identified in one passage, it was usually at P7. In general, there is an increase in the concentration of biomarkers with increasing conditioning time, which is higher after 48 h than after 24 h of conditioning. In OM-MSCs, the biomarkers Frutalkine, GRO/KC, LIX, and MCP-1 were only identified at passage P7; Rantes was only identified in P4, after 48 h of conditioning. Comparing the variation in the concentration of markers along the passages, it is possible to perceive that a large number of biomarkers are only identified in higher passages, P7, and whenever they are identified in P4 and P7, the concentration in P7 is higher. Likewise, the concentration of markers also increases within the same passage when the conditioning time is longer, with the highest concentrations observed in 48 h conditioning. All biomarkers that were identified in the CCM are shown in Table 5.

**Table 5. Mean normalized concentration of each biomarker in the CCM of OM-MSCs at P4 and P7(mean ± SD). nd = non-defined.**

| **Biomarker** | | **Mean normalized concentration** | | | |
|---|---|---|---|---|---|
| | | **P4 24h** | **P4 48h** | **P7 24h** | **P7 48h** |
| **EGF** | Mean | nd | nd | nd | nd |
| | SD | nd | nd | nd | nd |
| **Eotaxin** | Mean | nd | nd | nd | nd |
| | SD | nd | nd | nd | nd |
| **Fractalkine** | Mean | nd | nd | 0.040 | 0.040 |
| | SD | nd | nd | 0.00 | 0.00 |
| **G-CSF** | Mean | nd | nd | nd | nd |
| | SD | nd | nd | nd | nd |
| **GM-CSF** | Mean | nd | nd | nd | nd |
| | SD | nd | nd | nd | nd |
| **GRO/KC** | Mean | nd | nd | 0.08 | 0.19 |
| | SD | nd | nd | 0.00 | 0.00 |
| **IFN-γ** | Mean | 0.03 | 0.04 | 0.06 | 0.115 |
| | SD | 0.01 | 0.00 | 0.014 | 0.06 |
| **IL-1α** | Mean | nd | nd | nd | nd |
| | SD | nd | nd | nd | nd |
| **IL-1β** | Mean | nd | 0.46 | 0.67 | 0.84 |
| | SD | nd | 0.01 | 0.23 | 0.23 |
| **IL-2** | Mean | nd | nd | nd | nd |
| | SD | nd | nd | nd | nd |
| **IL-4** | Mean | nd | nd | nd | nd |
| | SD | nd | nd | nd | nd |
| **IL-5** | Mean | nd | nd | nd | nd |
| | SD | nd | nd | nd | nd |
| **IL-6** | Mean | nd | nd | nd | nd |
| | SD | nd | nd | nd | nd |
| **IL-10** | Mean | nd | 0.01 | 0.04 | 0.02 |
| | SD | nd | 0.01 | 0.01 | 0,00 |
| **IL-12p70** | Mean | nd | nd | nd | nd |
| | SD | nd | nd | nd | nd |
| **IL-13** | Mean | nd | nd | nd | nd |
| | SD | nd | nd | nd | nd |
| **IL-17A** | Mean | nd | nd | nd | nd |
| | SD | nd | nd | nd | nd |
| **IL-18** | Mean | nd | nd | nd | nd |
| | SD | nd | nd | nd | nd |
| **IP-10** | Mean | nd | 0.12 | 0.28 | 0.43 |
| | SD | nd | 0.01 | 0.00 | 0.04 |
| **Leptin** | Mean | nd | nd | nd | nd |
| | SD | nd | nd | nd | nd |
| **LIX** | Mean | nd | nd | 0.02 | 0.06 |
| | SD | nd | nd | 0.00 | 0.00 |
| **MCP-1** | Mean | nd | nd | 0.05 | 0.11 |
| | SD | nd | nd | 0.00 | 0.00 |
| **MIP-1α** | Mean | nd | nd | nd | nd |
| | SD | nd | nd | nd | nd |
| **MIP-2** | Mean | nd | nd | nd | nd |
| | SD | nd | nd | nd | nd |
| **RANTES** | Mean | nd | 0.01 | nd | nd |
| | SD | nd | 0.00 | nd | nd |
| **TNFα** | Mean | nd | nd | nd | nd |
| | SD | nd | nd | nd | nd |
| **VEGF** | Mean | 0.01 | 0.12 | 0.14 | 0.48 |
| | SD | 0.014 | 0.02 | 0.03 | 0.09 |

### Example 5. Effects of the use of mesenchymal stem cells from the olfactory mucosa and biomaterials in promoting peripheral nerve regeneration in the rat model

### 5.1 Determination of cytocompatibility between cells and biomaterials

The evaluation of cytocompatibility is essential to ensure that biomaterials do not induce cytotoxic effects on cells intended for regenerative applications. The interaction between OM-MSCs and Reaxon^{®} NGCs was assessed to determine their suitability for in vivo applications. By analysing cell viability in contact with the biomaterial, it is possible to confirm whether it provides a supportive environment for cell adhesion, proliferation, and survival. This validation is critical for the successful integration of OM-MSCs with Reaxon^{®} NGCs in peripheral nerve regeneration strategies.

### 5.1.1 In Vitro Cytocompatibility Assessment

To determine the compatibility between OM-MSCs and Reaxon^{®} Neural Guide Conduits (NGCs) to be used in the *in vivo* assays, a PrestoBlue^{™} viability assay was carried out. Cells were seeded in contact with Reaxon^{®} NGC and maintained in culture for 216 hours. At specific timepoints, cell viability was determined using the PrestoBlue^{®} viability assay, which confirms the cytocompatibility of a biomaterial when in contact with cells. Negative (standard culture medium) and positive (toxic agent - Dimethyl-sulfoxide (DMSO)) controls were used for comparison. Despite an initial delay, contact of cells with NGCs did not result in inhibition of cell proliferation over time, confirming the biocompatibility of the material which, after 216h, allowed cell viability identical to that observed in the negative control.

### 5.2 Scanning Electron Microscopy Analysis.

After determining the cytocompatibility between OM-MSCs and Reaxon^{®}, a scanning electron microscopy (SEM) Analysis and energy-dispersive X-ray spectroscopy (EDS) exams were performed. The evaluation of the samples through SEM allowed observing the external and internal architecture of the Reaxon^{®} NGCs, the morphology of OM-MSCs and the suitability of the biomaterial for cellular adhesion and proliferation, both individually and in homogeneous cell layers. The EDS analysis allowed confirming the presence of cells and homogeneous cell layers, namely, through the identification of nitrogen.

### In vivo assays

Thirty rats (Rattus norvegicus), Sasco Sprague-Dawley were used in this work. Rats submitted to a neurotmesis lesion of the sciatic nerve were divided into six experimental therapeutic groups : group 1 - uninjured control (UC); group 2 - group with end-to-end suture (ETE) 3 - suture of the nerve ends to the Reaxon^{®} neural guide tube NGC (R), group 4 - EtE and wrapping with Reaxon^{®} NGC (EtER), group 5 - suture of the nerve ends to the Reaxon^{®} NGC and administration of OM-MSCs suspended in Matrigel^{®} (ROM), and group 6 - EtE, wrapping with Reaxon^{®} NGC, and administration of OM-MSCs suspended in Matrigel^{®}. Nerve transection and repair were performed on the right hind limb, and the contralateral limb was used as the uninjured control. Primary outcomes consisted of assessing motor, nociceptive, behavioural function and kinematic evaluation over 20 weeks; secondary outcomes included the determination of the mass of the cranial tibial muscle and its histomorphometric evaluation and also the stereological assessment of the sciatic nerve after post-mortem tissue collection.

### 5.3 Functional Assessment

### 5.3.1 Motor Performance

To determine motor function, the animal's ability to exert weight on a scale was determined (Extensor Postural Test EPT). The force applied during contact with the weighing machine is measured in grams and recorded for both the healthy (NEPT) and injured (EEPT) limbs, determining the % motor deficit using the formula: $% motor deficit = \frac{NEPT - EEPT}{NEPT} \times 100 .$

After observing complete motor deficits (100%) resulting from the neurotmesis injury, there was a functional improvement over time. At 20 weeks, the ROM group was the one where the best result was observed, that is, the one where the motor function most closely resembled that observed in a healthy animal.

### 5.3.2 Nociceptive Function

The withdrawal reflex latency (WRL) test was used to assess the integrity of nociceptive function. The animal's limb is placed on a heating plate and the time it takes for the animal to remove it is counted, considering 12 seconds as the maximum time allowed. A healthy animal should withdraw the limb in 4.3 seconds or less. After a total absence of thermal sensitivity following nerve injury, a long-term recovery was observed with a decrease in WRL. After 20 weeks, the best result was observed in the EtER group, followed by the ROM group.

### 5.3.3 Walking Track Analysis

The assessment of the walking pattern was performed by two methods: sciatic functional index (SFI) and static sciatic index (SSI). In the first case, the rat walks along an acrylic corridor and its steps are filmed by a camera placed below the corridor. The SFI is calculated considering and through the elements print length (PL), toe spread distance between toes 1 and 5 (TS), and toe spread between toes 2 and 4 (ITS) and the formulas (N stands for "normal" and E stands for "experimental"): $Print lengh factor \left(PLF\right) = \frac{EPL - NPL}{NPL} ,$ $Toe spread factor \left(TSF\right) = \frac{ETS - NTS}{NTS} ,$ $Intermediate toe spread factor \left(ITF\right) = \frac{EIT - NIT}{NIT} .$ $SFI = \left(-38.3∗PLF\right) + \left(109.5∗TSF\right) + \left(13.3∗ITF\right) - 8.8 .$

SFI values are around 0 on healthy animals and close to -100 on animals with a complete lesion in the sciatic nerve.

In the second case, animal is kept inside an acrylic box, still, with the digital record being photographed at its base. The SSI is calculated using the formula: $Toe spread factor \left(TSF\right) = \frac{ETS - NTS}{NTS} ,$ $Intermediate toe spread factor \left(ITF\right) = \frac{EIT - NIT}{NIT} .$ $SSI = \left(108.44∗TSF\right) + \left(31.85∗ITF\right) - 5.49 .$

The SSI value is around -5 on healthy animals and around -90 on animals with complete sciatic nerve lesions.

Both SFI and SSI scores were low after nerve injury, with continuous and consistent recovery throughout the study period. After 20 weeks, the ROM group had the best results, followed by EtEROM.

### 5.3.4 Kinematic Analysis

Ankle kinematics during the gait cycle were recorded at the end of the 20 weeks for the established therapeutic groups and compared with the gait cycle pattern of the UC group, whose values were obtained before the induction of neurotmesis lesions. Using an acrylic corridor and filming cameras, the animal's gait was filmed to evaluate the stance phase and swing phase considered in the rat gait cycle, with positive and negative values corresponding to the position of the ankle joint indicate dorsiflexion and plantarflexion, respectively.

Regarding the spatiotemporal parameters of the gait, all groups presented longer cycle times, with an increased stance phase and swing phase. EtER and EtEROM revealed a similar pattern in terms of gait deviations when compared with the UC group, especially during the swing phase, with the EtEROM group presenting the best performance among the therapeutic groups.

**Table 6** contains a qualitative representation of the performance of the therapeutic groups in the different essays and tests performed *in vivo.* In general, it is possible to see that the groups that received OM-MSCs and NGCs had a better overall performance, with good results in basically all parameters.

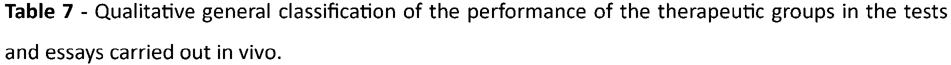

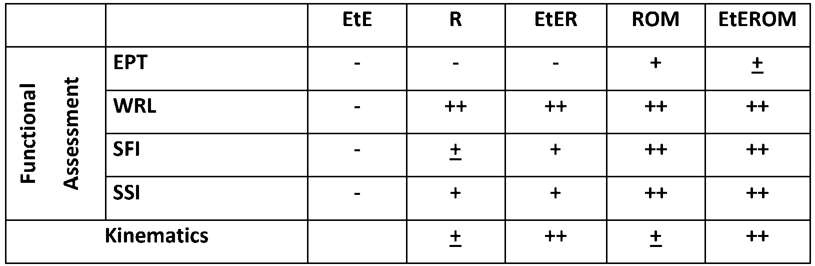

The groups were classified according to the statistical differences observed between them and the UC group: **** (-); *** (±); **, * (+); and no statistical differences (++).

### 5.4 Stereological and Histomorphometric Analysis

### 5.4.1 Nerve Stereological Analysis

After the established 20 weeks of the study, animals were euthanized, and the injured sciatic nerves and uninjured controls were collected and adequately fixed for further stereological evaluation through light and electron microscopy. The parameters total number of fibres (N), fibre density (N/mm2), axon diameter (µ), fibre diameter (µ), myelin thickness (M, µ), and cross-sectional area (mm2) were considered, in addition to the ratios d/D (g-ratio). All the therapeutic options considered demonstrated stereological evidence of nerve fibre regeneration with identical performances among all the therapeutic groups tested. However, the EtER and EtEROM groups showed evidence of more effective and advanced regeneration.

### 5.4.2 Histomorphometric Muscle Analysis

In parallel with the collection of the sciatic nerves, the cranial tibial muscles were also collected for further histomorphometric assessment and determination of the neurogenic atrophy degree, being weighed after harvest. Individual fibre measurements were obtained, and the muscle fibre area and minimal Feret's diameter (minimum distance of parallel tangents at opposing borders of the muscle fibre) were calculated. All cranial tibial muscles associated with sciatic nerve injury had lower final weights than the corresponding contralateral muscles at the time of collection. ROM and EtEROM groups had similar performances with the group with the best results in this assay, the group EtE.

The functional assessment, kinematic analysis, and histomorphometric evaluations confirmed that the use of Reaxon^{®} NGCs and OM-MSCs promoted functional improvements throughout the study period and evidence of nerve regeneration after 20 weeks, demonstrating promising effects of the use of OM-MSCs and biomaterials in promoting the regeneration of injured peripheral.

### Example 6. Effects of the use of the secretome of mesenchymal stem cells from the olfactory mucosa and biomaterials in promoting peripheral nerve regeneration in the rat model

### 6.1 In vivo assays

Thirty rats (Rattus norvegicus), Sasco Sprague-Dawley were used in this work. Rats submitted to a neurotmesis lesion of the sciatic nerve were divided into six experimental therapeutic groups (figure 30): group 1-Uninjured control (UC); group 2- EtE; group 3 - Suture of the nerve ends to the Reaxon^{®} nerve conduit and administration of OM-MSCs conditioned medium (CCM) (CMOM); group 4 - EtE, wrapping with Reaxon^{®} nerve conduit and administration of OM-MSCs CCM (ECMOM); group 5 - Suture of the nerve ends to the Reaxon^{®} nerve conduit and administration of olfactory ensheathing cells (OECs) CCM (CMOEC); group 6 - EtE, wrapping with Reaxon^{®} nerve conduit and administration of OECs CCM (ECMOEC). Reaxon^{®} NGCs with 15 mm long and 2.1 mm internal diameter were applied. Primary outcomes included the assessment of motor, nociceptive, and behavioural evolution over the study period; the secondary outcomes were based on the stereological evaluation of the sciatic nerve after harvesting and on the determination of the mass and histomorphometric characteristics of the cranial tibial muscle as an effector muscle of the sciatic nerve.

### 6.2 Motor Performance

Motor performance was determined as indicated above. Immediately after the neurotmesis injury, there was an evident loss of muscle strength in the hind limbs and a significant increase in the percentages of motor deficit in all intervened groups. Deficits gradually decreased over the study weeks. At the end of the study period, the CMOM group showed the lowest value of motor deficit.

### 6.3 Nociceptive Function

Nociceptive Function was determined as indicated above. After neurotmesis injury, a general increase in WRL time was observed in all therapeutic groups. At the end of the study period, the CMOM group was the one where better sensory recovery was observed.

### 6.4 Walking Track Analysis

Walking track analysis was performed as indicated above. After the neurotmesis injury, the functional consequences of the sciatic nerve injury translated into a significant impairment of the hind limb in all intervened groups. For the SFI, at the final timepoint, the CMOM group is the one with the best functional index values. Similarly, for SSI, CMOM group was again the one with the best performance at 20 weeks.

**Table 8** includes a qualitative description of the therapeutic groups' performance in the different functional assessment tests performed *in vivo.* In general, the groups that received CCM from both OM-MSCs and OECs had good performances in almost all parameters evaluated, with no marked statistical differences observed between the groups, but in absolute terms the CMOM group was the one that had the best overall performance.

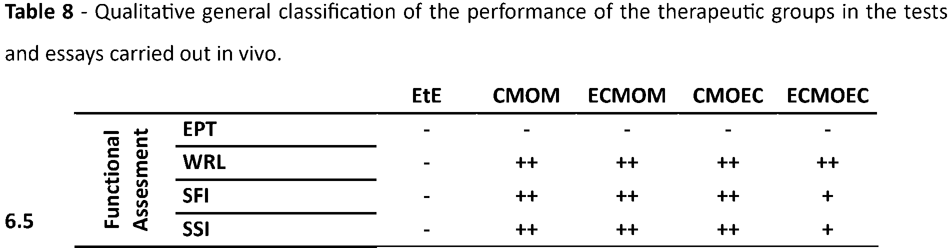

### Stereological and Histomorphometric Analysis

### 6.5.1 Nerve Stereological Analysis

As described above, after the established 20 weeks of the study, animals were euthanized, and the injured sciatic nerves and uninjured controls were collected and adequately fixed for further stereological evaluation through light and electron microscopy.

All the therapeutic options considered demonstrated the presence of microfasciculation phenomena, with the analysed nerves presenting a high number and density of fibres, fibres and axons of a small diameter, and thin myelin sheaths, observations expected in injured peripheral nerves that are undergoing a regeneration process. The groups that simultaneously received the biomaterials and OM-MSCs CCM tend to present better performance than those that received OECs CCM.

In parallel with the collection of the sciatic nerves, the cranial tibial muscles were also collected for further histomorphometric assessment and determination of the neurogenic atrophy degree, being weighed after harvest. The parameters evaluated were the same as those indicated above. All cranial tibial muscles associated with sciatic nerve injuries had a lower final mass than the contralateral healthy muscle. The group where a lower muscle loss was observed was the CMOM group.

In the fibre area evaluation, the highest final values were recorded in the groups CMOM and ECMOM. For the minimum Feret's angle parameter, the CMOM group is also among the best performing groups. The functional assessment, and histomorphometric evaluations confirmed that the use of Reaxon^{®} NGCs and OM-MSCs CCM promoted functional improvements throughout the study period and evidence of nerve regeneration after 20 weeks, demonstrating promising effects of the use of OM-MSCs CCM and biomaterials in promoting the regeneration of injured peripheral. The performance after using the cells or their CCM gave rise to similar results.

### Example 7. Effect of the combined use of Olfactory Mucosal Mesenchymal Stem Cells Secretome and Neural Guide Tubes for promoting nerve regeneration in the ovine model

Twelve sheep (*Ovis aries),* merino breed, female gender, 5 to 6 years and 50-60 kg BW were used in this work and subjected to a neurotmesis injury in the common peroneal nerve. After injury induction, three different therapeutic approaches were applied: (1) EtE suture; (2) application of NGC (NGC); (3) application of an NGC and secretome produced from OM-MSCs applied inside it (NGC- CCM), according to the described in **Alvites et. al (2025)** *.

The CCM used was produced as described previously. Nerve transection and repair were performed on the left hind limb, and the contralateral limb was used as UC. Primary outcomes consisted of performing a functional evaluation (mental status, gait, posture, and nociception), functional assessment, electromyography, and nerve and muscle ultrasound. Secondary outcomes consisted of a nerve stereological and muscle histomorphometric evaluation.

### 7.1 Functional Evaluation

After the induction of the lesions and the recovery of the animals, they were functionally evaluated through a neurological examination adapted to the common peroneal nerve and involving the evaluation of motor capacity through observation of movements and posture, proprioception through postural reactions and sensitivity through spinal reflexes. In each of the tests performed, the contralateral right limb was also tested as healthy control. Additionally, the mental state of the animals was also determined throughout the study. All animals were evaluated before the induction of the lesion to establish baseline values. After surgery, they were evaluated after one week (W1), after two weeks (W2) and from then on, every two weeks until week 24 (W24).

### 7.2 Posture

Posture was assessed with the animals in stationary position, observed in posterior and lateral views to determine the disposition and angulation between the different segments of the digits and the hook. The animals were classified on a scale of 1 to 5, with 1 corresponding to digits and hock in physiological position with no postural changes, and 5 to severe flexion of digits and extension of the hock, the expected manifestation of a common peroneal nerve injury. Immediately after induction of the neurotmesis injury, regardless of the therapeutic approach selected, all animals displayed the typical posture associated with injury to this nerve, namely overflexion of the distal joints and overextension of the hock, with the dorsal surfaces of the digits in contact with the ground during the stationary position. After 24 weeks of evaluation, the best performance was obtained in the EtE group, with the NGC and NGC- CCM groups presenting similar performance with no statistical differences between the therapeutic groups or with the UC group.

All animals maintained the ability to move freely throughout the monitoring period, performing free, voluntary and stimulated movements, without manifestations of pain or discomfort. At the initial timepoints, due to the direct consequences of the common peroneal nerve injury, during walking/running the animals dragged the limb with the dorsal surface of the digits in contact with the ground. As functional recovery was observed, the animals were able to recover normal posture during walking.

### 7.3 Postural reactions

Postural reactions were evaluated through proprioceptive assessment, which was determined through static and dynamic proprioceptive positioning tests. In the first case, the dorsal surface of the foot is placed in contact with the ground; in the second case, the foot is placed on a mobile platform that is slowly moved away from the animal, dragging the limb with it and altering the centre of balance. In both cases, the time in seconds that the animal takes to replace the limb to its physiological position is counted. The animals were classified on a scale of 1 to 5, being 1: <3 s; 2: 3-5 s; 3: 5-10 s; 4: 10-15 s; 5: 15-20 s; 6: >20 s. Immediately after the neurotmesis injury, the functional consequences dictated a total absence of proprioception of the intervened hind limb, with the animals maintaining the limb in the test position for at least 20s without repositioning it. At T24, all groups presented repositioning times very close to the considered normality (< 3 s), with no statistical differences between the study groups and the UC group, and with the NGC- CCM group presenting the best performance.

### 7.4 Spinal reflexes

Spinal reflexes were assessed using the withdrawal reflex, in which the animal, in lateral position and with the limb to be assessed facing upwards, and using hoof forceps, is stimulated by pinching the skin covering the dorsal surface of the lateral finger and the finger and hoof themselves. The animals were classified on a scale of 1 to 3, with 1 being the absence of a reflex, 2 the presence of a delayed reflex and 3 the presence of a normal reflex. Reflex identification includes the retraction of the limb after pinching, the manifestation of pain, and the animal's attempt to look at the stimulus location. Immediately after neurotmesis injury, an absence of sensitivity and of withdrawal reflex was observed in all considered groups. At T24, all groups presented sensitivity similar to that of healthy limbs, with a withdrawal reflex present and without response delays. Although at this final stage of evaluation the score observed in the therapeutic groups is lower than that observed in healthy animals, no statistical differences are observed between the groups and the NGC- CCM group is the one that presents the best performance.

### 7.8 Mental state

Throughout all tests, the animal's mental state was also assessed by determining the animal's ability to interact with the surrounding environment and the operator and to display typical species behaviour. No changes in mental status were identified in any animal during the evaluation period, and all sheep demonstrated their normal gregarious and environmental exploration behaviour, interacting with cohabiting animals and demonstrating awareness of the presence of operators. There was, therefore, no interference with the performance and results obtained in the neurological and functional evaluation examinations.

### 7.9 Kinematic evaluation

To evaluate gait characteristics, the animals were subjected to a kinematic assessment using a three-dimensional reconstruction of the hind limbs, based on gait spatiotemporal variables (namely flexion/extension, abduction/adduction and internal/external rotation), hip, knee and ankle angles and joint angle coordination measures during the walking cycles. All animals were evaluated before the induction of the lesion to determine the baselines values for kinematic analysis (W0). A new assessment was carried out after the study period (W24). The entire left hindquarter of the animal was trichotomized, from the thigh to the feet. An isolated room was prepared to allow the collection of images in a quiet and calm manner. A corridor was placed on the floor to facilitate the orientation of the operator and the animals during the walk. The animals were previously trained to move around the image collection room with an adapted leash, using concentrate food as a stimulus. Twenty reflective markers were placed on the hindlimb at specific anatomical locations to define the different bony segments. The animals were then encouraged to move around the image capture room, guided by a leash but in a way that did not limit their freedom of movement. The images were collected through 6 infrared cameras three placed on each side of the walking corridor, to identify the markers placed on both hindlimbs. Five body segments were reconstructed using Visual 3D software for biomechanics modelling: pelvis, thigh, leg, foot and metatarsus. Each segment has an embedded three-dimensional coordinate system. The hip joint angle, pelvis orientation, femur orientation, knee angle, and ankle joint angle were determined. Additionally, the spatiotemporal kinematic parameters cycle time, stance time, swing time, step length, step time, double limb support time and gait speed were calculated. Differences in gait curves were observed in all therapeutic groups compared to the control group. Overall, the results indicate that the ETE and NGC- CCM groups present more significant and broader changes in hip and knee movement patterns throughout the gait cycle, reflecting important functional impairments. In contrast, the NGC group demonstrates minimal or statistically non-significant differences regarding UC, suggesting a greater preservation of normal biomechanics. The application of NGCs led to better functional performance than the standard EtE suture. Likewise, the spatiotemporal analysis of the three groups revealed consistent patterns of gait asymmetry resulting from nerve injury, compatible with motor or neurological deficits and possible compensatory biomechanical mechanisms. In general, the right side was consistently used as a compensatory basis, while the left side, intervened, showed more signs of functional impairment. Considering the spatiotemporal parameters, the EtE group was the one where the final performance of the intervened limb was better at 24 weeks, but without unequivocal differences with the remaining therapeutic groups.

### 7.10 Ultrasound evaluation

To evaluate the characteristics of the common peroneal nerve, of the tibial cranial muscle and of the surrounding tissues after the induction of the lesion and application of the corresponding therapeutic approaches, an ultrasonographic evaluation was performed. All animals were evaluated before the induction of the lesion to determine the characteristics of the healthy nerve (W0). After surgery, they were evaluated after four weeks (W4), after twelve weeks (W12) and after 24 weeks (W24). Sheep were placed in recumbency on the surgical table, followed by preparation of the ultrasound field to be evaluated and by ultrasound identification of the common peroneal nerve and cranial tibial muscle. The ultrasound characteristics of each nerve and its relationship with neighbouring soft tissues were also evaluated. Subsequently, the ultrasound characteristics of each muscle and its relationship with neighbouring soft tissues were also evaluated.

After 1 month, the EtE group showed increased nerve diameter, oedema, and disorganized architecture. The NGC and NGC- CCM groups exhibited mild hypoechogenicity around the tubes and inflammatory exudate within their lumens. At 3 months, the EtE group displayed further enlargement with persistent disorganization, while the NGC and NGC- CCM groups showed early nerve continuity but incomplete fascicular organization and scar tissue formation. By 6 months, the EtE group had near-normal fascicular organization and reduced scarring. In the NGC groups, consolidated nerve regeneration was observed, with NGC- CCM showing a smaller nerve diameter compared to NGC but with both retaining visible scar tissue. Ultrasonographic evaluation showed an increase in nerve diameter at 1- and 3-months post-surgery in all groups, followed by a decrease at 6 months, most notably in the EtE and NGC- CCM groups, with NGC showing the largest final diameter. Statistically significant differences were observed between the UC and EtE groups at 1 month, and between the control group and all therapeutic groups at 3 and 6 months. No significant differences were found among the therapeutic groups throughout the study, with EtE showing the smallest final diameter, followed by NGC- CCM and NGC, all exceeding the diameter of healthy nerves.

Ultrasonographic analysis revealed significant changes in muscle structure over time in all groups compared to the UC group, which exhibited normal echogenicity and a well-defined fibrillar pattern.

At 1-month, acute denervation caused hypoechogenicity due to oedema and inflammation, masking the fibrillar pattern, most notably in the NGC- CCM group, followed by EtE. By 3 months, hyperechogenicity emerged, reflecting fibrous and fat infiltration and muscle degeneration, with the most disorganization in the EtE and NGC groups. At 6 months, muscles showed patterns closer to healthy tissue, with reduced infiltration and improved fibrillar organization, particularly in the NGC and NGC- CCM groups. At 1-month post-injury, all groups showed decreased muscle width and thickness, except for the NGC group, which maintained stable thickness throughout the study. By 3 months, the EtE group began recovering thickness, while the NGC- CCM group showed lower width and thickness compared to 1 month. At 6 months, both EtE and NGC- CCM groups exhibited increased thickness and width, with the NGC group showing the highest width and stable thickness near control values. The EtE group experienced the highest muscle atrophy at 1 month but showed strong recovery by 6 months. No significant statistical differences were observed among the groups or compared to UC at any timepoint.

### 7.11 Electrophysiological evaluation

To determine the level of reinnervation of the cranial tibial muscle as an effector organ of the common peroneal nerve, an electrophysiological evaluation was per-formed before surgical intervention to establish baseline values (W0), 4 (W4) and 12 (W12) weeks after surgery and at the end of the study period (24W). Sheep were placed in recumbency on the surgical table, and the procedure was performed on both the operated and healthy limbs. The common peroneal nerve was stimulated using stimulating bar electrodes placed on the skin over the nerve emergence site beneath the biceps femoris muscle, using an electromyography device. Monopolar recording needle electrodes were placed on the belly of the cranial tibial muscle (active electrode) and on its distal insertion tendon (reference). The ground electrode was placed proximally on the thigh. The compound muscle action potential was assessed by measuring the amplitude and latency at the maximal response observed. To this end, the intensity of the electrical stimulus was progressively increased until a maximum CMAP amplitude was obtained, associated with a contraction of the cranial tibial muscle and movement of the limb. Regarding amplitude, at 1-month post-injury, muscle contraction amplitude decreased in all therapeutic groups, with no response in the NGC and NGC- CCM groups, while the EtE group showed slightly higher values. Statistical differences were observed between the UC and all therapeutic groups. At 3 months, contraction amplitude improved in all groups, with EtE showing the best results and significant differences between UC and therapeutic groups, as well as between EtE and NGC. By 6 months, all groups showed further improvement, with NGC- CCM performing best and NGC worst, though values remained significantly lower than the UC group. Regarding latency, at 1-month post-injury, latency increased in the EtE group, while no muscle response was detected in the NGC and NGC-CCM groups. By 3 months, all groups showed muscle responses, with longer latency times in the NGC and NGC- CCM groups compared to EtE, which also showed a slight increase. Statistical differences were observed between the UC group and the guide tube groups. At 6 months, the NGC- CCM group showed a significant decrease in latency, reaching values like the EtE group, while the NGC group performed worst. No statistical differences with the UC group were observed at this time. Overall, the EtE group was the one that showed the best muscular response performance to electrical muscular stimulation after nerve injury and throughout the study period, with muscle response observed even after 1 month. However, after 6 months, the final performance of the NGC- CCM group was similar to that of the EtE group. At the last timepoint, the groups did not show differences regarding the UC group in determining latency, but these were observed regarding amplitude.

### 7.12 Nerve stereological analysis

After 24 weeks of study, the animals were euthanized and the common peroneal nerves were collected for later stereological evaluation, having been processed as previously described. After 24 weeks, all therapeutic groups showed stereological signs of nerve regeneration, including microfasciculation, higher fibre density, smaller axon and fibre diameters, and thinner myelin compared to the UC group. The EtE group had the best results for fibre and axon diameters, myelin thickness, and cross-sectional area, while the NGC group had the lowest values. Despite numerical differences, statistical significance was limited due to a small sample size. Significant differences with the UC group were observed for axon diameter, fibre diameter, and myelin thickness in the NGC group. The g-ratio was similar across groups, with no statistical differences.

### 7.13 Muscle histomorphometric Analysis

Simultaneously with nerve harvesting, the cranial tibial muscles were also collected for subsequent histomorphometric analysis and determination of the level of neurogenic atrophy. Muscle harvesting and processing was performed as previously described. For each individual fibre, the muscle fibre area and the minimum Feret's diameter (minimum distance of parallel tangents at opposing borders of the muscle fibre) were measured. A minimum of 200 fibres for each study group were analysed by two experienced operators. Regarding fibre area, among the therapeutic groups, the NGC group was the one that ended with a higher value, followed by NGC- CCM and EtE (Figure 53). In this case, statistical differences are always observed between the UC group and the therapeutic groups and also between NGC and the remaining groups. The minimum Feret's diameter results followed a similar organization, with NGC presenting the highest final value again, followed by NGC- CCM and EtE. The UC group presents statistically significant differences with EtE and NGC- CCM and also with NGC, but smaller in this case. NGC also presents differences with EtE. Comparing the final weight of the muscles associated with the injured nerve with those of the healthy limb within the same group, a percentage of muscle mass loss of 16.82% was observed for the EtE group, 6.95% for NGC and 21.84% for NGC- CCM. The NGC group, where the highest values of fibre area and minimum Feret's diameter were observed, was concordantly the group where the final muscle weight after the regeneration period was higher, and as such, where the loss of muscle mass was lower.

The results obtained in the in vivo and post-mortem evaluation after induction of neurotmesis injuries in the common peroneal nerves of the ovine model reveal that all therapeutic options could promote nerve and muscle regeneration effective enough to lead to functional recovery and evident histological and ultrastructural reorganization. However, contrary to what was previously observed in the rat model, the use of biomaterials and their combination with CCM did not always surpass the performance of traditional EtE techniques, and none of the therapeutic approaches were able to match the values recorded in healthy animals and tissues.

### REFERENCES

1. Zhu et al. (2024). Harnessing three-dimensional porous chitosan microsphere embedded with adipose-derived stem cells to promote nerve regeneration. Zhu et al. Stem Cell Research & Therapy (2024) 15:158
2. WO2023185379A1
   NERVE CONDUIT LOADED WITH ADIPOSE-DERIVED STEM CELLS AND PREPARATION METHOD THEREFOR
3. CN109224133A
   Preparation method of biological multilayer nerve conduit containing mesenchymal stem cells
4. Girard, S.D., Devéze, A., Nivet, E., Gepner, B., Roman, F.S., Féron, F. Isolating Nasal Olfactory Stem Cells from Rodents or Humans. J. Vis. Exp. (54), e2762, doi:10.3791/2762 (2011).
5. Tomé, M., Lindsay, S. L., Riddell, J. S., & Barnett, S. C. (2009). Identification of nonepithelial multipotent cells in the embryonic olfactory mucosa. Stem cells, 27(9), 2196-2208.
6. Ercolin, A. C. M., Roballo, K. C. S., Casals, J. B., Pieri, N. C. G., Souza, A. F., Barreto, R. D. S. N., ... & Ambrósio, C. E. (2016). Rabbit olfactory stem cells. Isolation protocol and characterization. Acta cirurgica brasileira, 31, 59-66.
7. Stamegna, J. C., Girard, S. D., Veron, A., Sicard, G., Khrestchatisky, M., Feron, F., & Roman, F. S. (2014). A unique method for the isolation of nasal olfactory stem cells in living rats. Stem cell research, 12(3), 673-679.
8. Alvites, R. D., Caseiro, A. R., Pedrosa, S. S., Branquinho, M. E., Varejão, A. S., & Maurício, A. C. (2018). The nasal cavity of the rat and mouse-source of mesenchymal stem cells for treatment of peripheral nerve injury. The Anatomical Record, 301(10), 1678-1689.
9. Alvites, R. D., Branquinho, M. V., Caseiro, A. R., Amorim, I., Santos Pedrosa, S., Rêma, A., ... & Maurício, A. C. (2020). Rat Olfactory Mucosa Mesenchymal Stem/Stromal Cells (OM-MSCs): A Characterization Study. International journal of cell biology, 2020(1), 2938258.
10. Alvites, R., Lopes, B., Coelho, A., Sousa, A. C., Sousa, P., Moreira, A., ... & Maurício, A. C. (2025). Combined Use of Olfactory Mucosal Mesenchymal Stem Cells Secretome and Neural Guide Conduits Promotes Nerve Regeneration in an Ovine Model. Frontiers in Cell and Developmental Biology. Not yet published

## Claims

1. A process for obtaining of mesenchymal stem cells (MSCs) from the olfactory mucosa **(OM-MSCs)** of a subject, comprising the following steps:
a) **Providing** a sample of olfactory mucosa (OM) of a given subject,
b) **Separation** of the mesenchymal stem cells **(OM-MSCs)** from the extracellular matrix of the olfactory mucosa (OM) of **a)** by a process comprising the following steps:
i) digestion with trypsin, and
ii) placing the OM fragments of **i)** in direct contact with the surface of a well-plate, leaving in rest for about 10-15 minutes without culture medium, at room temperature of 18 to 25ºC,
c) **Expanding** the isolated mesenchymal stem cells from the olfactory mucosa **(OM-MSCs)** of **b)** by culturing in a basal culture medium **(BCM)** comprising Dulbecco's modified eagle medium (DMEM/F12) having 10% Foetal Bovine Serum (FBS), 1.5% Penicillin/Streptomycin and 1.5% Amphotericin, and incubated under conventional conditions of CO₂ in humidified atmosphere, until confluency of 70-80% is achieved,
d) **Separation of** the secretome of mesenchymal stem cells **(OMS)** from the **OM-MSCs** of **c)** by:
i) collection and of the **OM-MSCs,**
ii) addition of trypsin to the separated **OM-MSCs** of **i),**
iii) centrifugation of the **OM-MSCs** of step **ii),** and
iv) Seeding the **OM-MSCs** of step **iii)** in fresh basal culture medium **(BCM)** under conditions of CO₂ in humidified atmosphere, until confluency of 70-80% is achieved.

2. A process of producing a conditioning culture medium **(CCM),** by culturing the mesenchymal stem cells from the olfactory mucosa **(OM-MSCs)** as described in claim 1 (iv) and by further removal of the basal culture medium (BCM) followed by addition of a specific culture medium **(DMEM)** comprising Dulbecco's modified eagle medium DMEM/F12 without antibiotic, antimycotic or FBS, being the resulting **OM-MSCs culture** further incubated under conventional conditions for +/- 48 h until confluency of 70-80% is achieved.

3. A process of obtaining OM-MSCs and the respective secretome (OMS) comprising the sequential cultivation of the isolated OM-MSCs as described in claim 1, for 9 passages (P0 to P9), each time by first replacing the incubated culture medium by fresh basal culture medium **(BCM)** and thereafter by conditioning the cultures of each stage by removal of the basal culture medium **(BCM)** followed by addition of **DMEM** culture medium, being the resulting **OM-MSCs culture** further incubated under conventional conditions for +/- 48h until confluency of 70-80% is achieved, thus defining a Conditioned Culture Medium **(CCM)** / secretome **(OMS)** corresponding to each of cultivation stages **P0 to P9.**

4. The conditioned culture medium (CCM) comprising OM-MSCs and the respective secretome (OMS) obtained by the process of any of the claim 1 to 3, **characterized by** presenting a chemokines and growth factors profile as described in the respective passages (P0 to P9) of Fig. 4.

5. The conditioned culture medium (CCM) according to claim 4, wherein the chemokines and growth factors profile is corresponding to stages P6 and P7, as described in Fig.4.

6. A medical device comprising the **OM-MSCs and/or the OMS** as described in any of the claims 4 or 5, wherein the **OM-MSCs and/or the OMS** are integrated in Neural Guide Conduits **(NGCs).**

7. A medical device according to **claim 6,** wherein the **NGCs** are chitosan-based NGCs having **OM-MSCs and/or the OMS** loaded microcarriers, NGCs incorporating **OM-MSCs and/or the OMS,** multilayer NGCs, electrospun nanofiber NGCs, hydrogel-based NGCs, biodegradable polymeric NGCs, or conductive NGCs.

8. A medical device according to claim 6 or 7, wherein the **OM-MSCs** corresponding to each of cultivation passages P0 to P9 of the conditioned medium (CCM) is integrated in Neural Guide Conduits (NGCs), thereby defining a **medical device A.**

9. A medical device according to claim 6 or 7, wherein the **secretome (OMS)** corresponding to each of cultivation passages P0 to P9 of the conditioned medium (CCM) is integrated in Neural Guide Conduits (NGCs), thereby defining a **medical device B.**

10. A medical device according to claim 6 or 7, wherein **OM-MSCs and** the respective secretome **(OMS)** are integrated in Neural Guide Conduits (NGCs), thereby defining a **medical device C.**

11. A medical device as described in any of the claims 6 to 10 to be applied in nerve tissue regeneration therapies.

12. A medical device as described in any of the claims 6 to 10 to be applied in peripheric nerve tissue regeneration therapies.
